(19) Europäisches Patentamt
European Patent Office
Office européen des brevets

(11) Veröffentlichungsnummer: **0 280 921**
**A2**

(12) # EUROPÄISCHE PATENTANMELDUNG

(21) Anmeldenummer: **88101753.7**

(22) Anmeldetag: **06.02.88**

(51) Int. Cl.4: **C07C 127/22**, A01N 47/34

(30) Priorität: **28.02.87 DE 3706524**

(43) Veröffentlichungstag der Anmeldung: **07.09.88 Patentblatt 88/36**

(84) Benannte Vertragsstaaten: **BE CH DE ES FR GB GR IT LI NL**

(71) Anmelder: **BASF Aktiengesellschaft**
**Carl-Bosch-Strasse 38**
**D-6700 Ludwigshafen(DE)**

(72) Erfinder: **Hofmeister, Peter, Dr.**
**Bernard-Humblot-Strasse 12**
**D-6730 Neustadt(DE)**
Erfinder: **Seppelt, Wolfgang, Dr.**
**Stettiner Strasse 1 a**
**D-6712 Bobenheim-Roxheim(DE)**
Erfinder: **Harries, Volker, Dr.**
**Immengartenweg 29 e**
**D-6710 Frankenthal(DE)**

(54) **[N-Benzoyl-N'-(3,5-dichlor-4-phenoxy)-phenyl]-harnstoffe.**

(57) [N-Benzoyl-N'-(3,5-dichlor-4-phenoxy)-phenyl]-harnstoffe der Formel,

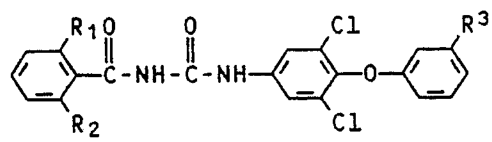

in der die Substituenten folgende Bedeutung haben:

$R^1$ Fluor oder Chlor
$R^2$ Wasserstoff, Chlor oder Brom und
$R^3$ Fluor oder Brom,

Verfahren zu ihrer Herstellung und ihre Verwendung zur Bekämpfung von Schädlingen.

EP 0 280 921 A2

## [N-Benzoyl-N'-(3,5-dichlor-4-phenoxy)-phenyl]-harnstoffe

Die vorliegende Erfindung betrifft neue [N-Benzoyl-N'-(3,5-dichlor-4-phenoxy)-phenyl]-harnstoffe der allgemeinen Formel I

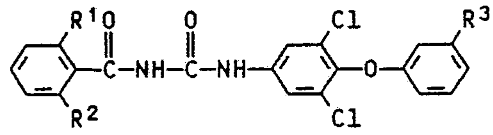

(I)

in der die Substituenten folgende Bedeutung haben:

$R^1$ Fluor oder Chlor,
$R^2$ Wasserstoff, Fluor oder Chlor und
$R^3$ Fluor oder Brom.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus der EP-A-101,990 sind Verbindungen des Typs I bekannt, die sich dahingehend unterscheiden, daß sie im Substituenten $R^3$ Wasserstoff, Chlor oder Trifluormethyl bedeuten. Die Wirkung dieser Verbindungen war vor allem bei niedrigen Aufwandmengen nicht voll befriedigend.

Der Erfindung lag daher die Aufgabe zugrunde, neue [N-Benzoyl-N'-(3,5-dichlor-4-phenoxy)-phenyl]-harnstoffe mit verbesserter Wirkung bereitzustellen.

Demgemäß wurden die eingangs definierten neuen [N-Benzoyl-N'-(3,5-dichlor-4-phenoxy)-phenyl]-harnstoffe I, Verfahren zu deren Herstellung, Schädlingsbekämpfungsmittel, die die Verbindungen I als Wirkstoffe enthalten, sowie ein Verfahren zur Bekämpfung von Schädlingen mit [N-Benzoyl-N'-(3,5-dichlor-4-phenoxy)-phenyl]-harnstoffen I gefunden.

Bevorzugt sind Verbindungen I, in denen $R^1 = R^2 =$ Fluor, $R^1 =$ Fluor und $R^2 =$ Chlor, $R^1 =$ Chlor und $R^2 =$ Wasserstoff und $R^1 = R^2 =$ Chlor ist.

Die Verbindungen I sind nach folgenden Methoden erhältlich:

a) durch Umsetzung eines Benzoylisocyanates der allgemeinen Formel II

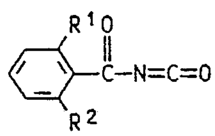

(II)

mit einem (3,5-Dichlor-4-phenoxy)-anilin der allgemeinen Formel III

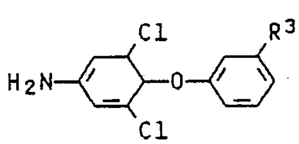

(III)

oder

b) durch Umsetzung eines Benzamides der allgemeinen Formel IV

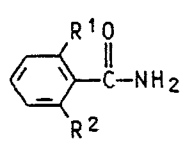

(IV)

mit einem (3,5-Dichlor-4-phenoxy)-phenylisocyanat der allgemeinen Formel V

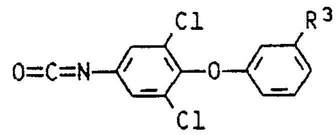

(V).

Die Umsetzung a) verläuft bei Temperaturen von 0 bis 120°C, vorzugsweise 20 bis 80°C und besonders bevorzugt bei 20 bis 60°C, sowie bei Drücken zwischen 1 und 10 bar, vorzugsweise bei Normaldruck. Da die Reaktion unter Wärmeentwicklung (exotherm) verläuft, kann es von Vorteil sein, eine Kühlmöglichkeit vorzusehen.

Zweckmäßigerweise wird das (3,5-Dichlor-4-phenoxy)-anilin der Formel III in einem Lösungs-oder Verdünnungsmittel vorgelegt unter anschließender Zugabe des Benzoylisocyanats der Formel II. Die Reaktion ist im allgemeinen nach 2 Stunden beendet.

Die Benzoylisocyanate II sind bekannt oder nach bekannten Methoden herstellbar (J. Org. Chem. 28, (1963), 1805-1811; J. Agr. Chem. 21, (1973), 348-354). Die (3,5-Dichlor-4-phenoxy)-aniline III sind gleichfalls bekannt oder nach bekannten Methoden herstellbar (Organikum, 9. Auflage, 1969, S. 446, 567, VEB Deutscher Verlag der Wissenschaften).

Die Umsetzung b) wird gegebenenfalls in Gegenwart eines Katalysators, bei Temperaturen von 0 bis 140°C, vorzugsweise zwischen 60 und 100°C, sowie bei Drücken zwischen 1 und 10 bar, vorzugsweise bei Normaldruck, durchgeführt. Die Reaktionsdauer liegt im allgemeinen zwischen 2 und 6 Stunden.

Als Katalysatoren eignen sich beispielsweise organische Basen, wie Triethylamin, Pyridin, 4-N,N-Dimethylaminopyridin oder Alkylmetallverbindungen, wie Dibutylzinndiacetat,

Die Benzamide IV sind bekannt oder nach bekannten Methoden (z.B. Houben-Weyl, Methoden der organischen Chemie, Bd. E 5.2, S. 934 ff, Georg-Thieme Verlag, 1985) erhältlich. Die (3,5-Dichlor-4-phenoxy)-phenylisocyanate V sind z.T. bekannt oder sie können nach bekannten Methoden (z.B. Houben-Weyl, Methoden der organischen Chemie, Bd. E 4, S. 738 ff, Georg Thieme Verlag, 1983) erhalten werden.

Üblicherweise setzt man die Ausgangsstoffe II und III bzw. IV und V in stöchiometrischem Verhältnis ein. Ein Überschuß der einen oder anderen Komponente kann in Einzelfällen aber durchaus vorteilhaft sein.

Die (3,5-Dichlor-4-phenoxy)aniline III und die Isocyanate V können mit üblichen Methoden aus entsprechenden Nitrobenzolen durch Reduktion hergestellt werden (Houben-Weyl, Methoden der organischen Chemie, Band XI/I, (1975), S. 360 ff). Die entsprechenden Nitrobenzole lassen sich z.B. nach Barry et al., Pr. Irish Acad. 53B, (1950), 6166-6182 herstellen.

Die Umsetzungen a) und b) werden zweckmäßigerweise in einem Lösungs-oder Verdünnungsmittel ausgeführt. Hierzu sind beispielsweise geeignet; aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan und Chlorbenzol, Ether oder Ester wie Diethyl-und Di-n-Butylether, Methyl-tert.-butylether, Tetrahydrofuran, Dioxan und Essigsäureethylester; Ketone, beispielsweise Aceton, Methylethylketon und Methylisopropylketon; ferner Nitrile, wie Acetonitril und Propionitril; aprotische dipolare Lösungsmittel, wie Dimethylformamid, Dimethylsulfoxid oder Pyridin. Auch Gemische dieser Stoffe können als Lösungs-oder Verdünnungsmittel verwendet werden.

Die neuen Verbindungen der Formel I fallen teilweise in Form von Ölen an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Die [N-Benzoyl-N'-(3,5-dichlor-4-phenoxy)-phenyl]-harnstoffe der Formel I sind geeignet, Schädlinge aus der Klasse der Insekten und Spinntiere wirksam zu bekämpfen. Sie können im Pflanzenschutz sowie auf dem Hygiene-, Vorratsschutz-und Veterinärsektor als Schädlingsbekämpfungsmittel eingesetzt werden.

Ein besonders wichtiges Anwendungsgebiet der erfindungsgemäßen Mittel sind Raupen von Schadschmetterlingen. Daneben eignen sich die beanspruchten [N-Benzoyl-N'-(3,5-dichlor-4-phenoxy)-phenyl]-harnstoffe aufgrund ihrer stark chemosterilisierenden Eigenschaften vor allem sehr gut zur Bekämpfung von Dipteren, Spinnmilben und Schaben.

Zu den schädlichen Insekten gehören aus der Ordnung der Schmetterlinge (Lepidoptera) beisielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu-und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana

(Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleneule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius califonicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuermeise), Atta sexdens (Blattschneiderameise);

aus der Ordnung der Walzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina) beispielsweise Ixodes ricinus (Holzbock), Ornithodorus moubata, Amblyomma americanum, Dermacentor silvarum, Boophilus microplus, Bryobia praetiosa.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatsiche, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoren, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentration, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier-oder Emulgiermittel in Wasser homogenisiert werden. Es können aber auch aus wirksamer Substanz Netz-, Haft-, Dispergier-oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali-und Erdalkalisalze der Dibutylnaphthalilnsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu-und Stäubemittel können durch Mischen oder gemeinsames Vermahlen der wirksamen Substanzen mit einem festen Trägerstoff hergestellt werden.

Beispiele für Formulierungen sind:

I. 5 Gewichtsteile der Verbindung Nr. 1 werden mit 95 Gewichtsteilen feinteiligem Kaolin innig vermischt. Man erhält auf diese Weise ein Stäubemittel, das 3 Gew.% des Wirkstoffs enthält.

II. 30 Gewichtsteile der Verbindung Nr. 6 werden mit einer Mischung aus 92 Gewichtsteilen pulverförmigem Kieselsäuregel und 8 Gewichtsteilen Praffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 1 werden in einer Mischung gelöst, die aus 90 Gewichtsteilen Xylol, 6 Gewichtsteilen des Anlagerungsprodukte von 8 bis 10 Mol Ethlyenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 6 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenxid an 1 Mol Ricinusöl besteht.

V. 80 Gewichtsteile der Verbindung Nr. 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Granulate, z.B. Umhüllungs-, Imprägnierungs-und Homogengranulate können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium-und Magnesiumsulfat, Magnesiumoxid, gemahlene Kunststoffe,

Düngemittel, wie z.B. Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver und andere feste Trägerstoffe.

Bevorzugte Formulierungen sind Emulsions-und Suspensionskonzentrate, die im allgemeinen zwischen 0,1 und 95 Gew.% Wirkstoff, vorzugsweise zwischen 0,5 und 90 Gew.% Wirkstoff enthalten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen z.B. 0,01 bis 3, vorzugsweise 0,02 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden:

1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Isopropoxyphenyl-N-methylcarbamat, 3,5-Dimethyl-4-met hylmercapto-phenyl-N-methylcarbamat, 4-Dimethylamino-3,5-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-0-(methylcarbamoyl)-oxim, S-Methyl-N-[(methylcarbamoyl)-oxy]thio-acetimidat, Methyl-N',N'-dimethyl-N-[(methylcarbamoyl)oxy]-1-thiooxamidat, N-(2-Methyl-chlor-phenyl)-N',N'dimethylformamidin, Tetrachlorthiophen, 1-(2,6-Difluor-benzoyl)-3(4-chlor-phenyl)-harnstoff, 0,0-Dimethyl-0-(pnitrophenyl)-phosphorthioat, 0,0-Diethyl-0-(p-nitrophenyl)-phosphorthioat, 0-Ethyl-0-(p-nitrophenyl)-phenyl-phosphonothioat, 0,0-Dimethyl-0-(3-methyl-4-nitrophenyl)-phosphorthioat, 0,0-Diethyl-0-(2,4-dichlorphenyl)-phosphorthioat, 0-Ethyl-0-(2,4-dichlorphenyl)phenyl-phosphonothioat, 0,0-Dimethyl-0-(2,4,5-trichlorphenyl)-phosphorthioat, 0-Ethyl-0-(2,4,5-trichlorphenyl)-phosphorthioat, 0,0-Dimethyl-0-(2,5-dichlor-4-jodphenyl)-phosphorthioat, 0,0-Dimethyl-0-(3-methyl-4-methylthiophenyl)-phosphorthioat, 0-Ethyl-0-(3-methyl-4-methylthiphenyl)-isopropyl-phosphoramidat, 0,0-Diethyl-0-[p-methylsulfinyl-phenyl]-phosphorthioat, 0-Ethyl-S-phenyl-ethyl-phosphonodithioat, 0,0-Diethyl-[2-chlor-1-(2,4-dichlorphenyl)-vinyl]phosphat, 0,0-Dimethyl-[-2-chlor-1-(2,4,5-trichlorphenyl)]-vinyl-phosphat, 0,0-Dimethyl-S-(1'-phenyl)-ethylacetat-phosphordithioat, Bis-(dimethylamino)-fluorphosphinoxid, Octamethyl-pyrophosphoramid, 0,0,0,0-Tetraethyldithio-pyrophosphat, S-Chlormethyl-0,0-diethyl-phosphordithioat, 0-Ethyl-S,S-dipropylphosphor-dithioat, 0,0-Dimethyl-0-2,2-dichlorvinyl-phosphat, 0,0-Dimethyl-1,2-dibrom-2,2-dichlorethylphosphat, 0,0-Dimethyl-2,2,2-trichlor-1-hydroxy-ethylphosphonat, 0,0-Dimethyl-S-[1,2-bis-carbethoxy-ethyl-(1)]-phosphordithioat, 0,0-Dimethyl-0-(1-methyl-2-carbmethoxy-vinyl)-phosphat, 0,0-Dimethyl-S-(N-methyl-carbamoyl-methyl)-phosphordithioat, 0,0-Dimethyl-S-(N-methyl-carbamoyl-methyl)phosphor thioat, 0,0-Dimethyl-S-(N-methoxyethyl-carbamoyl-methyl)-phosphordithioat, 0,0-Dimethyl-S-(N-formyl-N-methyl-carbamoylmethyl-phosphordithioat, 0,0-Dimethyl-0-[(1-methyl-2-(methyl-carbamoyl)-vinyl]phosphat, 0,0-Dimethyl-0-[(1-methyl-2-dimethylcarbamoyl)-vinyl]phosphat, 0,0-Dimethyl-0-[(1-methyl-2-chlor-2-diethylcarbamoyl)-vinyl]-phosphat, 0,0-Diethyl-S-(ethyl-thio-methyl)-phosphordithioat, 0,0-Diethyl-S-[(p-chlor-phenylthio)-methyl]-phosphordithioat, 0,0-Dimethyl-S-(2-ethylthioethyl)-phosphorthioat, 0,0-Dimethyl-S-(2-ethylthioethyl)-phosphordithioat, 0,0-Diethyl-S-(2-ethylthio-ethyl)-phosphordithioat, 0,0-Diethyl-S-(2-ethylsulfinyl-ethyl)-phosphorthioat, 0,0-Diethyl-thiophosphoryliminophenyl-acetonitril, 0,0-Diethyl-S-(2-chlor-1-phthalimidoethyl)-phosphordithioat, 0,0-Diethyl-S-[6-chlor-benzoxazolon-(2)-yl(3)]-methyldithiophosphat, 0,0-Dimethyl-S-[2-methoxy-1,3,4-thiadiazol-5-[4H]-onyl-(4)-methyl]-phosphordithioat, 0,0-Diethyl-0-[3,5,6-trichlor-pyridyl-(2)]-phosphorthioat, 0,0-Diethyl-0-(2-pyrazinyl)-phosphorthioat, 0,0-Diethyl-0-[2-isopropyl-4-methyl-lpyrimidinyl(6)]-phosphorthioat, 0,0-Diethyl-0-[2-(diethylamino)-6-methyl-4-pyrimidinyl]-thionophosphat, 0,0-Dimethyl-S-(4-oxo-1,2,3-benzotriazin-3-[4H]-yl-methyl)-phosphordithioat, 0,0-Dimethyl-S-[(4,6-diamino-1,3,5-triazin-2-yl)-methyl]-phosphordithioat, 0,0-Diethyl-(1-phenyl-1,2,4-triazol-3-yl)-thionophosphat, 0,S-Dimethyl-phosphor-amidothioat, 0,S-Dimethyl-N-acetyl-phosphoramidothioat, alpha-Hexachlorcyclohexan, 1-1-Di-(p-methoxyphenyl)-2,2,2-trichlor-ethan, 6,7,8,9,10,10-Hexachloro1,5,5a,6,9,9a-hexahydro-6,9-methano-2,4,3-benzodi oxa-thiepin-3-oxid, Pyrethrine, DL-2-Allyl-3-methyl-cyclopenten-(2)-on-(1)-yl-(4)-DL-cis, trans-chrysanthemat, 5-Benzyl-furyl-(3)-methyl-DL-cis, trans-chrysanthemat, 3-Phenoxybenyl(±)-cis, trans-2,2-dimethyl-3-(2,2--dichlorvinyl)-cyclopropancarboxylat, alpha-Cyano-3-phenoxy-benzyl(±)-cis, trans-2,2-dimethyl-3-(2,2-dichlorvinyl)-cyclopropancarboxylat, (s)-alpha-Cyano-3-phenoxybenzyl-cis(1R,3R),-2,2-dimethyl-3-(2,2-dibromvinyl)--cyclopropancarboxylat, 3,4,5,6-Tetrahydrophthalimidoethyl-DL-cis,trans-chrysanthemat, 2-Methyl-5-(2-propinyl)--3-furylmethyl-chrysanthemat, (alpha-Cyano-3-phenoxybenzyl)-alpha-isopropyl-4-chlorphenylacetat.

Herstellungsbeispiel

[N-2,6-Difluorbenzoyl-N'-{3,5-dichlor-4-(3-bromphenoxy)}-phenyl]-harnstoff (Beispiel 6)

219,7 g (1,27 mol) 3-Bromphenol werden in 1 Liter Dimethylformamid vorgelegt. Man setzt 104 g (1,3 mol) konzentrierte Natronlauge nach und nach bei Raumtemperatur (20-30°C) zu und trägt 344,2 g (1,27 mol) 3,5-Dichlor-4-bromnitrobenzol ein. Nach Rühren über Nacht bei Raumtemperatur (20-30°C) wird noch 1 Stunde auf 50°C erhitzt, die Reaktionsmischung anschließend in 5 l Eiswasser gegossen und 2 Stunden nachgerührt. Das ausfallende Produkt wird abgesaugt, mit Wasser gewaschen und bei 50°C i. Vak. getrocknet. Man erhält 453,6 g (98,4 %) 3,5-Dichlor-4-(3-bromphenoxy)-nitrobenzol vom Fp. 90 bis 95°C.

23,0 g (0,063 mol) des zuvor erhaltenen Produktes werden in 150 ml wasserfreiem Tetrahydrofuran gelöst, mit 2,5 g Raney-Nickel versetzt und bei Raumtemperatur unter einem Druck von 0,1-0,3 bar 5 Stunden hydriert. Man erhält 3,5-Dichlor-4-(3-bromphenoxy)-anilin in nahezu quantitativer Ausbeute, Fp. 99 bis 100°C.

Zu einer Lösung von 7,0 g (0,021 mol) des zuvor erhaltenen Produktes in 50 ml wasserfreiem Tetrahydrofuran tropft man bei Raumtemperatur 4,2 g (0,023 mol) 2,6-Difluorbenzoylisocyanat. Anschließend wird 2 Stunden bei 45°C gerüht und nach Abkühlen auf 0°C mit 100 ml n-Pentan versetzt und abgesaugt. Man erhält 9,17 g (84,5 %) [N-2,6-Difluorbenzoyl-N'-{3,5-dichlor-4-(3-bromphenoxy)}-phenyl]-harnstoff vom Fp. 200 bis 201°C.

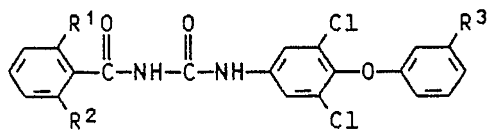

(I)

| Verb. Nr. | $R^1$ | $R^2$ | $R^3$ | Fp.[°C] |
|---|---|---|---|---|
| 1 | F | F | F | 174-175 |
| 2 | F | H | F | |
| 3 | F | Cl | F | |
| 4 | Cl | Cl | F | |
| 5 | Cl | H | F | 177-178 |
| 6 | F | F | Br | 200-201 |
| 7 | F | H | Br | |
| 8 | F | Cl | Br | |
| 9 | Cl | Cl | Br | |
| 10 | Cl | H | Br | 193-194 |

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen.

A: 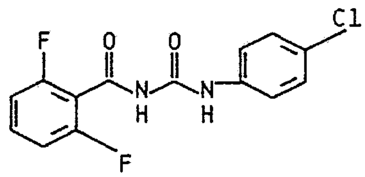 bekannt aus DE-A-21 23 236 als Anspruch 69

B: 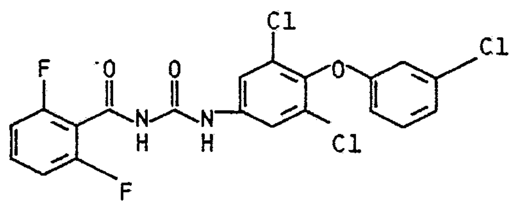 bekannt aus EP-A-101 990 als Verbindung Nr. 17

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Beispiel 1

Musca domestica (Stubenfliegen), Chemosterilisation

Ca. 100 schlüpffähige Puppen von Stubenfliegen werden in Folienbehälter mit 8,5 l Volumen gesetzt. Sie erhalten hier 2 g Futter, bestehend aus

6 Teilen Kristallzucker
6 Teilen Trockenmilch
1 Teil Eipulver

dem zunächst 20 mg des Wirkstoffes in organischer Lösung zugefügt wurden (= 1 Gew.%). Wasser wird in ausreichender Menge auf Zellstoff in 100 ml Bechern geboten. Die Tiere bleiben ca. 8 Tage in diesen Käfigen bei 22°C und Tag-und Nachtrhythmus. Am. 8. Tag gibt man ein Eiablagegefäß mit in Magermilch getränktem Zellstoff in die Käfige. Hier erfolgt innerhalb der nächsten 24 Stunden die Eiablage. Beim Einstellen der Eiablagegefäß wird die Fraßgiftwirkung anhand der toten Fliegen beurteilt. Nach 3 bis 4 Stunden wird die Entwicklung der Eier auf die Milchwatte beurteilt.

| Verbindung Nr. | Konzentration [%] | Mortalität [%] |
|---|---|---|
| 1 | 0,004 | 80 |
| 6 | 0,002 | 80 |
| | 0,004 | 100 |
| A | 0,4 | 100 |
| B | 0,02 | 100 |

Beispiel 2

Tetranychus telarius (Spinnmilbe), Versuche mit gezählten Weibchen

Getopfte Buschbohnen werden in der Spritzkabine auf dem Drehteller mit der wäßrigen Wirkstoffaufbereitung tropfnaß gespritzt. Nach dem Abtrocknen stanzt man mit einem Korkbohrer Blattstücke von 25 mm Durchmesser aus den Spreiten. Sie werden auf Zellstoffstücke gelegt, welche an den Seiten in Wasser hängen. Die Blattstanzstücke werden mit je 10 adulten Weibchen belegt. Nach 5 und 10 Tagen erfolgt die Auswertung, wobei Eier, Larven und Adulte ausgezählt werden.

| Verbindung Nr. | Konzentration [%] | Hemmung [%] |
|---|---|---|
| 1 | 0,1 | ca. 40 |
| 6 | 0,04 | ca. 80 |
| A | 0,1 | 0 |
| B | 0,1 | 0 |

Beispiel 3

Blatta orientalis (Schabe), Zuchtversuch

Die Prüfung erfolgt in 1 l-Gläsern an 50 Schaben im ersten Larvenstadium. Ein Gestell aus 5 horizontalen Pappscheiben (6 × 6 cm) bietet den Tieren Schlupfmöglichkeit. Der Wirkstoff wird über das Futter aufgenommen. Dieses besteht aus 20 g kalt angerührtem Karottenbrei in Petrischalen (Ø 5 cm). Zusätzlich erhalten sie Wasser auf Zellstoff in Plastikbechern (Inhalt 25 ml). Die Beobachtungszeit erstreckt sich über zwei Häutungsphasen (3 Wochen). Dabei wird wöchentlich mit frisch zubereitetem Brei nachgefüttert. Die Beurteilung erfolgt wöchentlich.

| Verbindung Nr. | Konzentration [ppm] | Mortalität [%] |
|---|---|---|
| 1 | 40 | ca. 16 |
| 6 | 10 | 100 |
| A | 10 | 100 |
| B | 100 | 0 |

Beispiel 4

Plutella maculipennis (Kohlschabe), Zuchtversuch

Kohlpflanzen mit einem Tag alten Eiablagen von Plutella maculipennis werden in der Spritzkabine mit der wäßrigen Aufbereitung des Wirkstoffes tropfnaß gespritzt. Nach dem Abtrocknen stellt man die Pflanzen unter transparente Kunststoffhauben mit Gazefenster. Nach Bedarf werden unbehandelte Kohlpflanzen dazugestellt. Der Versuch läuft bis zum Schlüpfen der Falter. Bei der Beurteilung vergleicht man die Populationsentwicklung mit der unbehandelten Kontrolle.

| Verbindung Nr. | Konzentration [ppm] | Hemmung [%] |
|---|---|---|
| 1 | 0,02 | ca. 80 |
| 6 | 0,01 | ca. 90 |
| A | 0,1 | 0 |
| B | 0,2 | ca. 90 |

Beispiel 5

Prodenia litura (Ägypt. Baumwollwurm), Entwicklungshemmung auf behandeltem Nährboden

Die Zucht erfolgt in 250 ml Bechern auf ca. 100 ml des Standard-Nährbodens, dem in flüssigem Zustand der Wirkstoff sorgfältig untergemischt wurde. Je Konzentration wird 1 Becher mit 5 Raupen im dritten Larvenstadium (10-12 mm Länge) angesetzt. Die Gefäße werden mit perforierten, transparenten Deckeln verschlossen. Die Beobachtung erstreckt sich bis zum Schlüpfen der Falter. Die Versuchstemperatur beträgt 25-26°C.

| Verbindung Nr. | Konzentration [ppm] | Mortalität [%] |
|---|---|---|
| 1 | 1 | ca. 78 |
| 6 | 1 | 100 |
| | 40 | 100 |
| A | 4 | ca. 94 |

Beispiel 6

Sitophilus granaria (Kornkäfer), Zuchtversuch

1 kg Weizen wird mit 1 bzw. 2 g eines wirkstoffhaltigen Talkum-Staubes bzw. einer Wirkstoffkombination innig gemischt. Darauf füllt man 100 g des Getreides in kleine Flaschen, belegt diese mit 200 Kornkäfer, verschließt die Flaschen mit Pappscheiben und lagert sie bei 20 bis 22°C. Nach 14 Tagen werden die Kornkäfer abgesiebt, die Zahl der toten Tiere wird bestimmt und das Getreide weiter in den verschlossenen Flaschen bis zum Schlüpfen der Jungtiere in der unbehandelten Kontrolle bei 20 bis 22°C gelagert (ca. 8 Wochen). Darauf wird das Getreide wieder gesiebt und die Zahl der Tiere pro Glas festgestellt und verglichen.

| Verbindung Nr. | Konzentration [ppm] | Mortalität [%] |
|---|---|---|
| 1 | 10 | ca. 80 |
| 6 | 20 | ca. 80 |
| B | 100 | ca. 85 |

## Ansprüche

1. [N-Benzoyl-N'-(3,5-dichlor-4-phenoxy)-phenyl]-harnstoff der Formel I

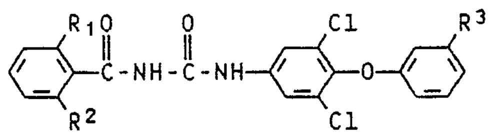

(I),

in der die Substituenten folgende Bedeutung haben:
$R^1$ Fluor oder Chlor,
$R^2$ Wasserstoff, Fluor oder Chlor und
$R^3$ Fluor oder Brom.

2. Verfahren zur Herstellung von [N-Benzoyl-N'-(3,5-dichlor-4-phenoxy)-phenyl]-harnstoffen der allgemeinen Formel I gemäß Anspruch 1, dadurch gekennzeichnet, daß man

a) ein Benzoylisocyanat II

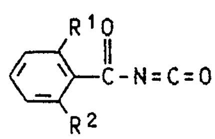

(II)

mit einem (3,5-Dichlor-4-phenoxy)-anilin III

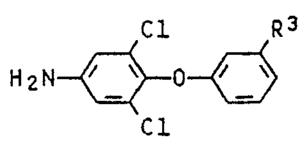

(III)

oder

b) ein Benzamid IV

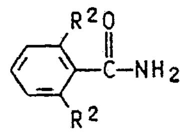

(IV)

mit einem Isocyanat V

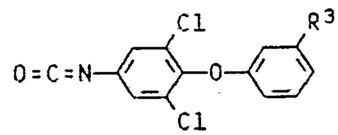

(V)

in an sich bekannter Weise umsetzt.

3. Schädlingsbekämpfungsmittel, enthaltend einen [N-Benzoyl-N'-(3,5-dichlor-4-phenoxy)-phenyl]-harnstoff der Formel I gemäß Anspruch 1 neben üblichen Trägerstoffen.

4. Schädlingsbekämpfungsmittel, gemäß Anspruch 3 dadurch gekennzeichnet, daß es 0,1 bis 95 Gew.% eines [N-Benzoyl-N'-(3,5-dichlor-4-phenoxy)-phenyl]-harnstoffes I enthält.

5. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines [N-Benzoyl-N'-(3,5-dichlor-4-phenoxy)-phenyl]-harnstoffes der Formel I gemäß Anspruch 1 behandelt.

Patentansprüche für folgenden Vertragsstaat : ES

1. Verfahren zur Herstellung von [N-Benzoyl-N'-(3,5-dichlor-4-phenoxy)-phenyl]-harnstoff der allgemeinen Formel I

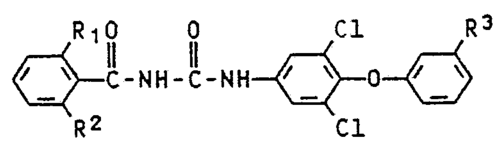

(I),

in der die Substituenten folgende Bedeutung haben:
$R^1$ Fluor oder Chlor,
$R^2$ Wasserstoff, Fluor oder Chlor und
$R^3$ Fluor oder Brom,

dadurch gekennzeichnet, daß man
a) ein Benzoylisocyanat II

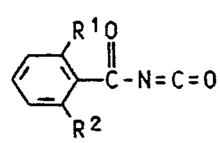

(II)

mit einem (3,5-Dichlor-4-phenoxy)-anilin III

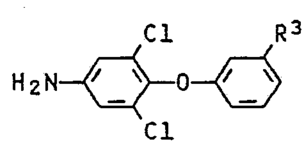

(III)

oder
b) ein Benzamid IV

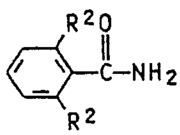

(IV)

mit einem Isocyanat V

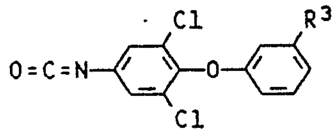

(V)

in an sich bekannter Weise umsetzt.

2. Schädlingsbekämpfungsmittel, enthaltend einen [N-Benzoyl-N'-(3,5-dichlor-4-phenoxy)-phenyl]-harnstoff der Formel I gemäß Anspruch 1 neben üblichen Trägerstoffen.

3. Schädlingsbekämpfungsmittel, gemäß Anspruch 2, dadurch gekennzeichnet, daß es 0,1 bis 95 Gew.% eines [N-Benzoyl-N'-(3,5-dichlor-4-phenoxy)-phenyl]-harnstoffes I enthält.

5. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines [N-Benzoyl-N'-(3,5-dichlor-4-phenoxy)-phenyl]-harnstoffes der Formel I gemäß Anspruch 1 behandelt.